(19) Europäisches Patentamt
European Patent Office
Office européen des brevets

(11) Veröffentlichungsnummer: **0 030 394**
**B1**

(12) **EUROPÄISCHE PATENTSCHRIFT**

(45) Veröffentlichungstag der Patentschrift: **10.08.83**

(51) Int. Cl.³: **C 07 C 69/157**, C 07 C 67/00

(21) Anmeldenummer: **80200899.5**

(22) Anmeldetag: **25.09.80**

(54) **Verfahren zur Herstellung von Arylestern von Carbonsäuren.**

(30) Priorität: **11.12.79 DE 2949671**

(43) Veröffentlichungstag der Anmeldung: **17.06.81 Patentblatt 81/24**

(45) Bekanntmachung des Hinweises auf die Patenterteilung: **10.08.83 Patentblatt 83/32**

(84) Benannte Vertragsstaaten: **DE FR GB IT NL**

(56) Entgegenhaltungen:
**EP-A-0 019 304**

**„Journal of the American Society", Bd. 92, Nr. 11, 3. Juni 1970, S. 3520-3522, Washington, USA, „Thallium in organic synthesis. XV. Synthesis of phenols and aromatic nitriles".**

(73) Patentinhaber: **Rütgerswerke Aktiengesellschaft, Mainzer Landstrasse 217, D-6000 Frankfurt a.Main 1 (DE)**

(72) Erfinder: **Knips, Ulrich, Dr., Im Baunhof 1, D-5970 Plettenberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 030 394 B1

ACTORUM AG

# Verfahren zur Herstellung von Arlyestern von Carbonsäuren

Die Erfindung betrifft ein Verfahren zur Herstellung von Carbonsäurearylestern durch katalytische Zersetzung der entsprechenden Monoarylthalliumsalze unter Erhaltung des Substitutionsmusters des Arylrestes nach Art einer Dethallierungsreaktion.

Durch Umsetzung von aromatischen Kohlenwasserstoffen mit Thallium(III)salzen sind Monoarylthalliumsalze in hoher Isomerenreinheit erhältlich, wobei das Anion des Thalliumsalzes sowohl anorganischer als auch organischer Natur sein kann.

Aus „J. Am. Soc.", *92* (1970), S. 3520-3522 ist bekannt, ein derartiges Monoarylthalliumsalz, das Arylthalliumdi(trifluoracetat) zu Aryltrifluoracetat umzusetzen und dieses anschliessend zum Phenol zu hydrolysieren. Die Umsetzung zum Aryltrifluoracetat erfolgt mit Hilfe von Bleitetraacetat in Tetrahydrofuranlösung mit anschliessender Triphenylphosphinzugabe und Ausscheidung der Blei- und Thalliumionen mittels HCl.

Die allgemeine Nutzung von Arylthalliumsalzen zur Gewinnung von Carbonsäureestern in einem vereinfachten Verfahren ist Gegenstand der EP-A Nr. 0019304. Danach werden Monoarylthallium(III)salze der allgemeinen Formel

$$Ar - TlXY,$$

worin Ar sowohl ein- oder mehrkernige Arylreste als auch ein- oder mehrkernige Heteroarylreste, X und Y sowohl identische Carboxylatreste als auch verschiedene Carboxylatreste oder X einen Carboxylatrest und Y einen Säurerest einer starken Säure bedeuten, in wässeriger Lösung in Gegenwart katalytischer Mengen von Palladiumsalzen unter milden Bedingungen umgesetzt und die erhaltenen Arylester von der wässerigen Phase in bekannter Weise abgeschieden oder mit Lösungsmitteln extrahiert. Als Nebenprodukt werden die entsprechenden symmetrischen Biaryle beobachtet.

Die Produktverteilung liegt im besten Falle (p-Tolylthalliumacetatperchlorat) bei einem Gewichtsverhältnis der beiden Reaktionsprodukte von 86,1% Arylester zu 13,9% Biaryl, was einer 79%igen Selektivität der Esterbildung entspricht. Die Umsatzrate der Arylthalliumsalze in die Reaktionsprodukte Arylester und Biaryl liegt nach 2 h insgesamt bei 74-75,1%. Das erforderliche molare Verhältnis von Palladium zu Thallium beträgt 1:150.

Der vorliegenden Erfindung lag die Aufgabe zugrunde, die Monoarylthalliumsalze für die technische Gewinnung von Carbonsäurearylester nutzbar zu machen, indem versucht werden sollte, den Anteil des erfindungsgemäss darstellbaren Arylesters zuungunsten des weniger interessanten Biaryls zu erhöhen, die Ausbeute bzw. die Umsatzrate des einzelnen Produktionsschrittes zu erhöhen und die erforderliche Menge des Palladiumkatalysators weiter zu verringern.

Die gestellte Aufgabe wird erfindungsgemäss gelöst durch ein Verfahren zur Herstellung von Arylestern von Carbonsäuren durch Umsetzung von in wässeriger Lösung vorliegenden Monoarylthallium(III)salzen der allgemeinen Formel

$$Ar - TlXY,$$

worin Ar sowohl ein- oder mehrkernige Arylreste als auch ein- oder mehrkernige Heteroarylreste, X und Y sowohl identische Carboxylatreste als auch verschiedene Carboxylatreste oder X einen Carboxylatrest und Y einen Säurerest einer starken Säure bedeuten, in Gegenwart katalytischer Mengen von Palladiumsalzen unter milden Bedingungen und Abtrennung der gebildeten Arylester in bekannter Weise durch Kristallisation oder Destillation, dadurch gekennzeichnet, dass die Palladiumsalze in einem mit Wasser nicht mischbaren Lösungsmittel gelöst vorliegen und die Umsetzung in Form einer Zweiphasenreaktion unter Mischen erfolgt.

Es wurde gefunden, dass grundsätzlich jedes Monoarylthalliumsalz, das nach bekannten Methoden darstellbar ist, für die erfindungsgemässe Umsetzung geeignet ist, bevorzugt jedoch die Arylthallium(III)perchlorat(carboxylate), da sie in reinem säurefreiem Wasser eine sehr gute Löslichkeit ohne nennenswerte Hydrolyse- oder Zerfallserscheinungen besitzen. Diese Monoarylthalliumsalze bilden Carbonsäurearylester in wässerigen Lösungsmitteln wie 5-50%iger Carbonsäure, vorzugsweise jedoch in reinem Wasser. Der Carbonsäurezusatz empfiehlt sich in den Fällen, wo die eingesetzten Arylthalliumsalze zur Hydrolyse neigen, wie z.B. die Trifluoroacetate. Die Reaktion wird bei Normaldruckverhältnissen, bei Temperaturen zwischen 20 und 100° C durchgeführt, vorzugsweise bei 40-70° C, in Gegenwart katalytischer Mengen von Palladiumsalzen, die in einem nicht mit Wasser mischbaren Medium gelöst sind.

Als besonders gut in nichtwässerigen Solvenzien lösliche Palladiumverbindungen sind Palladium(II)salze organischer Säuren, wie z.B. die niedriger aromatischer Carbonsäuren, besonders jedoch aliphatischer Carbonsäuren wie z.B. Palladium(II)acetat geeignet, ausserdem die Palladiumsalze von Mineralsäuren, sofern eine ausreichende Löslichkeit in dem zur Verwendung gelangenden nichtwässerigen Lösungsmittel besteht, wie z.B. Palladium(II)chlorid. Die Palladiumsalze müssen im allgemeinen bei der erfindungsgemässen Verfahrensführung in einem molaren Verhältnis Palladium zu Thallium von 1:200 bis 1:400 vorliegen. Noch niedrigere Palladiumkonzentrationen bewirken zwar ebenfalls den erfindungsgemässen Ablauf des Verfahrens, doch verzögern sich die Reaktionszeiten beträchtlich.

Als Lösungsmittel für die Palladiumsalze sind insbesondere aromatische Kohlenwasserstoffe geeignet, z.B. Benzol, Toluol, Äthylbenzol, Kumol, o-Xylol, m-Xylol, p-Xylol, Mesitylen oder Gemische dieser Stoffe, aber auch Alkane und Cycloalkane, soweit das Lösungsvermögen für die erforderliche Palladiumsalzmenge ausreicht.

Das Verfahren wird dergestalt durchgeführt, dass die wässerige Lösung der Arylthalliumsalze und die Katalysatorlösung vereinigt und damit zur Reaktion gebracht werden, was vorteilhaft durch kräftiges Rühren des Zweiphasengemisches erreicht wird.

Die gebildeten Carbonsäureester und die als Nebenprodukt anfallenden Biaryle scheiden sich mit dem nichtwässerigen Medium ab, woraus sie vorteilhaft durch Destillation oder Kristallisation nach bekannten Methoden in reiner Form erhalten werden können.

Die bei dem patentgemässen Verfahren erreichbare Selektivität der Umsetzung der Arylthalliumsalze zu den Carbonsäureestern kann praktisch den optimalen Wert von 100% erreichen. So entsteht z.B. beim Einsatz des 2,4- und 2,5-Xylylthalliumacetatperchlorats das entsprechende Acetoxixylol jeweils mit einer Selektivität von 98% bei einem Umsatzgrad von 93 bzw. 98% nach 3 h Reaktionszeit. p-Tolylacetat ist unter sonst gleichen Bedingungen nach 2 h Reaktionszeit bei 99% Umsatz mit einer Selektivität von 95% darstellbar. Die Selektivität wird entscheidend durch die Anwesenheit freier Carbonsäuren und — in geringerem Masse — freier Mineralsäuren beeinträchtigt.

Die nach dem Verfahren herstellbaren Carbonsäurearylester besitzen eine Bedeutung als Vorstufen der Phenole durch Hydrolyse nach bekannten Methoden. Darüberhinaus besitzen einige, insbesondere der Essigsäure-p-tolylester, eine Bedeutung als Duftstoffe in der Parfümindustrie. Vorteilhaft einsetzbar sind Carbonsäurearylester bei chemischen Synthesen, wie durch die als Fries-Umlagerung bekannte Darstellung von o- und p-Hydroxiacetophenonen aus Essigsäurearylestern belegt wird.

*Beispiel 1*

Eine Lösung von 1000 Gew.-Teilen p-Tolylthalliumacetat(perchlorat) $p\text{-}CH_3C_6H_4TlClO_4(OAc) \cdot H_2O$ in 6000 Gew.-Teilen Wasser und eine Lösung von 2 Gew.-Teilen Palladium(II)acetat $Pd(OAc)_2$ in 1500 Gew.-Teilen Benzol wird 2 h lang bei 60° C kräftig verrührt. Anschliessend wird die organische Phase abgetrennt und bei vermindertem Druck das Benzol abdestilliert. Es bleiben 310 Gew.-Teile eines Rückstandes zurück, der neben Palladiumverbindungen 297 Gew.-Teile p-Tolylacetat und 11 Gew.-Teile 4,4'-Dimethylbiphenyl enthält. Die Umsatzrate beträgt 99% bei einer Selektivität von 95%. Das Gemisch wird über eine kurze Kolonne bei 10 mbar rektifiziert, wobei die zwischen 79 und 85° C übergehende Fraktion aus reinem p-Tolylacetat besteht. Der Destillationsrückstand wird mit Aceton aufgenommen und daraus das 4,4'-Dimethylbiphenyl durch Wasserzugabe in reiner Form gefällt.

*Beispiele 2-4*

1000 Gew.-Teile des Xylylthalliumacetat-(perchlorats) werden in 6000 Gew.-Teilen Wasser gelöst und mit einer Lösung von 2 Gew.-Teilen $Pd(OAc)_2$ in 1500 Gew.-Teilen Benzol 3 h lang kräftig verrührt. Die organische Phase wird abgetrennt, das Benzol abdestilliert und der verbleibende Rückstand bei vermindertem Druck zur Gewinnung des Essigsäurexylylesters rektifiziert. Aus dem Destillationsrückstand ist das entsprechende symmetrische Tetramethylbiphenyl nach Lösung mit Aceton und Fällung durch Wasser in reiner Form zu gewinnen.

Tabelle der Ergebnisse gemäss obiger Beschreibung

| Beispiel | Xylylrest | Xylylacetat Gew.-Teile | Tetramethylbiphenyl Gew.-Teile | Umsatz nach 3 h (%) | Selektivität (%) |
|---|---|---|---|---|---|
| 2 | 3,4- | 236 | 26 | 82 | 85 |
| 3 | 2,4- | 307 | 4 | 91 | 98 |
| 4 | 2,5- | 324 | 4 | 98 | 98 |

*Beispiel 5*

1000 Gew.-Teile p-Äthylphenylthalliumacetat-(perchlorat) werden entsprechend den Beispielen 1-4 zur Reaktion gebracht. Nach 2 h Reaktionszeit fallen 291 Gew.-Teile eines Gemisches an, das aus 276 Gew.-Teilen p-Äthylphenylacetat und 15 Gew.-Teilen 4,4'-Diäthylbiphenyl besteht. Bei einem Umsatzgrad von 89% beträgt die Selektivität der Esterbildung 92%.

## Patentansprüche

1. Verfahren zur Herstellung von Arylestern von Carbonsäuren durch Umsetzung von in wässeriger Lösung vorliegenden Monoarylthallium(III)salzen der allgemeinen Formel

$$Ar - TlXY,$$

worin Ar sowohl ein- oder mehrkernige Arylreste als auch ein- oder mehrkernige Heteroarylreste, X und Y sowohl identische Carboxylatreste als auch verschiedene Carboxylatreste oder X einen Carboxylatrest und Y einen Säurerest einer starken Säure bedeuten, in Gegenwart katalytischer Mengen von Palladiumsalzen unter milden Bedingungen und Abtrennung der gebildeten Arylester in bekannter Weise durch Kristallisation oder Destillation, dadurch gekennzeichnet, dass die Palladiumsalze in einem mit Wasser nicht mischbaren Lösungsmittel gelöst vorliegen und die Umsetzung in Form einer Zweiphasenreaktion unter Mischen erfolgt.

2. Verfahren nach Anspruch 1, dadurch gekennzeichnet, dass die Arylthalliumsalze in reinem Wasser gelöst werden.

3. Verfahren nach Anspruch 1, dadurch gekennzeichnet, dass die Arylthalliumsalze in 5-50%iger wässeriger Lösung einer wasserlöslichen Carbonsäure gelöst werden.

4. Verfahren nach den Ansprüchen 1 und 3, dadurch gekennzeichnet, dass als wässerige Carbonsäurelösung eine Lösung der Carbonsäure verwendet wird, deren Arylester erhalten werden sollen, wobei Arylthalliumsalze der allgemeinen Formel

$$Ar - Tl(CF_3-COO)_2$$

eingesetzt werden.

5. Verfahren nach den Ansprüchen 1 bis 3, dadurch gekennzeichnet, dass man als Monoarylthalliumsalze solche der allgemeinen Formel

$$Ar-TlX\ ClO_4$$

zur Reaktion bringt.

6. Verfahren nach den Ansprüchen 1 bis 5, dadurch gekennzeichnet, dass die Umsetzung bei 40-70° C unter normalem Druck innerhalb von 2-4 h durchgeführt wird.

## Claims

1. Process for the preparation of aryl esters of carboxylic acids by reacting an aqueous solution of monoarylthallium(III)salts of the general formula

$$Ar - TlXY,$$

wherein Ar is selected from the group consisting of mono- or polynuclear aryl radicals and mono- and polynuclear heteroaryl radicals, X and Y are either identical carboxylate radicals or different carboxylate radicals, or X is a carboxylic radical and Y is an acid radical of a strong acid, in the presence of catalyc amounts of palladium salts under mild conditions and separation by known methods of the aryl esters formed by cristallization or distillation, characterized in that the palladium salts are dissolved in a water-immiscible solvent and the reaction takes place in the form of a two-phase reaction with mixing.

2. The process of claim 1, characterized in that the aryl thallium salts are dissolved in pure water.

3. The process of claim 1, characterized in that the aryl thallium salts are dissolved in an aqueous solution of 5-50% by weight of a water soluble carboxylic acid.

4. The process of the claims 1 and 3, characterized in that the aqueous carboxylic acid solution is a solution of the carboxylic acid from which aryl esters can be obtained and the aryl thallium salts are of the general formula

$$Ar - Tl(CF_3 - COO)_2$$

5. The process of the claims 1 to 3, characterized in that monoaryl thallium salts of the general formula

$$Ar - TlX\ ClO_4$$

are reacted.

6. The process of the claims 1 to 5, characterized in that the reaction is carried out within 2-4 h at temperatures in the range of 40-70° C at normal pressure.

## Revendications

1. Procédé de préparation d'esters aryliques d'acides carboxyliques par conversions de sels de monoarylthallium(III) en solution aqueuse, de formule générale

$$Ar - TlXY,$$

dans laquelle Ar désigne un radical arylique ou hétéroarylique, monocyclique ou polycyclique, et X et Y désignent des radicaux carboxylates identiques ou différents l'un de l'autre, ou bien X représente un radical carboxylate et Y le radical acide d'un acide fort, en présence de quantités catalytiques de sels de palladium, dans des conditions douces et avec séparation, de manière connue, des esters aryliques formés, par cristallisation ou distillation, procédé caractérisé en ce que les sels de palladium sont employés en solution dans un solvant non miscible à l'eau, et la conversion se fait sous la forme d'une réaction en deux phases, avec mélange.

2. Procédé selon la revendication 1, caractérisé en ce que les sels d'arylthallium sont dissous dans de l'eau pure.

3. Procédé selon la revendication 1, caractérisé en ce que les sels d'arylthallium sont dissous dans une solution aqueuse à 5-50% d'un acide carboxylique hydrosoluble.

4. Procédé selon l'une des revendications 1 à 3, caractérisé en ce que la solution aqueuse est une solution de l'acide carboxylique dont on veut obtenir l'ester arylique, et les sels d'arylthallium sont des sels de formule

$$Ar - Tl\ (CF_3 - COO)_2$$

5. Procédé selon l'une des revendications 1 à 3, caractérisé en ce que les sels de monoarylthallium sont des sels de formule générale

$$Ar - TlClO_4$$

6. Procédé selon l'une des revendications 1 à 5, caractérisé en ce que la réaction est exécutée en 2 à 4 h entre 40 et 70° C, à la pression normale.